# EUROPEAN PATENT APPLICATION

(11) **EP 1 023 882 A1**
(43) Date of publication of application: **02.08.2000**
(21) Application number: 00100779.8
(22) Date of filing: 15.01.2000
(51) Int. Cl.: A61F 5/44, A61M 25/00, A61M 25/01

(54) **A urinary catheder assembly**

(30) Priority: 26.01.1999 DK 2599
(71) Applicant: COLOPLAST A/S, 3050 Humlebaek (DK)
(72) Inventor: Pedersen, Jens Kristian, 3100 Hornbaek (DK); Jensen, Lars Boegelund, 2300 Koebenhavn S (DK)
(74) Representative: Nilausen, Kim (DK)

(57) **Abstract**

A urinary catheter assembly comprising a urinary catheter (1) and a flexible tubular catheter package comprising a hose member (4) with a cavity narrowly surrounding the catheter (1). The hose member (4) is provided with elongation means, preferably in the form of click-clack corrugations.

## Description

### FIELD OF THE INVENTION

The present invention relates to a urinary catheter assembly comprising a urinary catheter and a flexible tubular catheter package comprising a hose member with a cavity narrowly surrounding the catheter.

### BACKGROUND OF THE INVENTION

Urinary catheters of the kind contemplated by the present invention are increasingly used for so-called intermittent catheterization of the bladder. A typical use is for post-operative urine retention of newly operated patients in hospitals, for whom intermittent catheterization performed with intervals of 3 to 6 hours has brought a significantly reduced risk of infection of the urethra and the bladder compared to permanent catheterization.

Another typical use is with patients suffering from severe cases of urinary incontinence as for disabled individuals like para- or tetraplegics who frequently have no control permitting voluntary urination.

For such users intermittent catheterization has become increasingly common also in daily life situations outside the clinical environment of a hospital, whereby a significantly improved quality of life has been obtained for this group of patients.

For many such users it is necessary, however, to connect the catheter with a urine collection bag through a hose connected, at one end, with the bag and, at the other end, with the terminal member of the catheter, with the inherent disadvantage that several connecting operations must be performed prior to use of the catheter. Furthermore for such users to dispose the collected urine afterwards, it is normally necessary to disconnect the assembly and/or operating closing valves.

To overcome this problem it is known, e.g., from GB-A-2,284,764, US-A-2,856,932, US-A-4,379,506, US-A-4,204,527, US-A-4,246,909, WO 94/06377, WO 97/26937 and Danish Design Registration No. 0932-1986 to integrate the catheter with the urine collection bag, typically by arranging the catheter inside the bag combined with a bag design permitting partly withdrawal of the catheter from the bag to provide a projecting catheter of a length sufficient for insertion through the urethra into the bladder.

Whereas catheter-bag combinations of this kind have undoubtedly facilitated the use of intermittent catheterization, they have not remedied the disadvantage associated with disposal of the collected urine.

In a disposable male urethral catheter assembly known from US-A-4,246,909 the catheter-bag combination is made into a single integrated unit which is disposable after use. A catheter having an enlarged or bulbous discharge end is contained in a sterile environment in an upper chamber of a flexible bag. The upper chamber is detachably connected at one end with a lower sample chamber for collection of a urine sample and is partly defined at the opposite end by two chevrons defining an opening which is reduced in size relative to the enlarged discharge end of the catheter.

In use the penis is inserted into an open top of the bag outside the two chevrons and by manipulation of the upper chamber without touching the catheter the latter may be inserted into the urethra, until the enlarged discharge end contacts the opening defined by the two chevrons, whereby the upper chamber forming a short extension in flow communication with the catheter will direct a urine sample flowing through the catheter to the lower sample chamber.

This disposable solution suffers, however, from significant cost disadvantages. With this background, it is the object of the invention to provide a simple and relatively inexpensive catheter set permitting disabled users, even when wheel chair bound, to drain the urine flow through the catheter directly into a toilet or other available drainage means, leaving only the catheter and its package as items which must be disposed after use.

### BRIEF DESCRIPTION OF THE INVENTION

The present invention relates to a urinary catheter assembly comprising a urinary catheter and a flexible tubular catheter package comprising a hose member with a cavity narrowly surrounding the catheter.

### BRIEF DESCRIPTION OF THE DRAWINGS

In the following the invention will be explained in further detail with reference to the accompanying drawings, in which
Fig. 1 is a longitudinal sectional view of an embodiment of a catheter assembly according to the invention in its supply condition;
Fig. 2a is a longitudinal sectional view of the hose member of a second embodiment of a catheter assembly according to the invention before elongation and
Fig. 2b is a hose member in its longitudinal sectional view of the in its elongated form;
Fig. 3 is a longitudinal sectional view of the hose member of a third embodiment of a catheter assembly according to the invention, and
Fig. 4 is a longitudinal sectional view of a fourth embodiment of a catheter assembly according to the invention.

The drawings are all schematic examples of specific embodiments of the invention and are not to be considered as being limiting for the scope of the invention which is defined by the appended claims.

### DETAILED DESCRIPTION OF THE INVENTION

The invention relates to a urinary catheter assembly comprising a urinary catheter 1 and a flexible tubular catheter package comprising a hose member 4 with a cavity narrowly surrounding the catheter 1, said assembly being characterised in that at least part of the hose member 4 is provided with elongation means.

The advantage of this embodiment is that the catheter assembly in its stored condition takes up very little room and is therefore handy to carry along. When the catheterization takes place, the catheter package acts as an extension for the catheter in order to drain the urine flow through the catheter directly into a toilet or other available collection or drainage means without spillage and leaving only the catheter and its package as items which must be disposed after use. The hose member of the catheter package is elongated by activating the elongation means before the catheterization takes place.

The catheter package therefore defines a substantially longer extension for the catheter than provided for by the state of the art. As the catheter package in stored condition has substantially the same length as the catheter itself one may understand that the option of elongation is especially an advantage in connection with female catheters, as they are of approximately half length compared to male catheters and as the distal end of the catheter is less accessible after insertion than in a male.

In a preferred embodiment of a urinary catheter assembly of the invention, the elongation means is provided in the form of click-clack corrugations. Click-clack corrugations are corrugations with sharp edges as illustrated in the drawings. Click-clack corrugations are well known when used for production of, e.g., straw. The tube provided with click-clack corrugations may be bent without essentially narrowing the opening thereof, and the tube may be elongated simply by stretching the corrugations and it may be shortened by simply pushing the ends together re-establishing the corrugations.

When the tubular catheter package with the click-clack corrugations according to the present invention is elongated the corrugations will enable the catheter package to stay in the position given by the user during the catheterization and until the user changes the position, e.g., after the catheterization the user pushes the catheter package back to initial position by pressing the elongation means together. With click-clack corrugations the elongation is very easy to perform even for users with poor dexterity.

In a further preferred embodiment of the catheter assembly of the invention at least part of the hose member is provided with soft corrugations. When the hose member of the catheter package is provided with soft corrugations, it makes the catheter assembly more flexibly. It is an advantage when the catheter assembly is packed, stored and especially when it is carried along by the user in some kind of bag.

In a still further preferred embodiment the urinary catheter assembly of the invention, a first end of the catheter 1 is connected with a connector member 3. The package further comprises a tubular compartment 10 connected with hose member 4 for accommodation of the connector member 3. The compartment 10 is closed by a first closure 11, whereas in a second, opposite end it is connected with the hose member 4. A second end of the hose member 4 remote from its connection with the compartment 10 is closed by a second closure 7. The connector member 3 is adapted for connection with the second end of the hose member 4 after removal of said first closure 11 and the second closure 7, so that when removing the catheter 1 from the hose member 4 the latter forms an extension member in flow communication with the catheter 1.

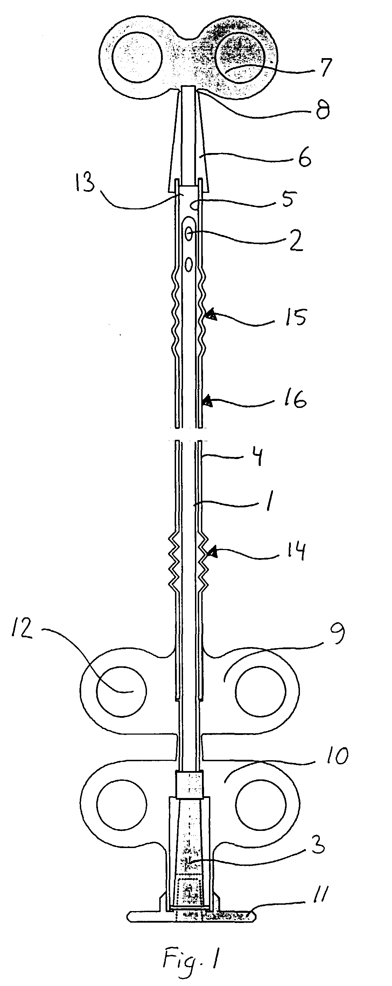

The catheter assembly shown in Fig. 1 comprises an elongated urinary catheter 1 having a first end, at which one or more inlet openings 2 for inflow of urine are provided. In its opposite end the catheter 1 is preferably inseparably connected with a slightly conical connector member 3, which may be used for connecting of the catheter with a standard hose connector for a drainage hose for the purpose of draining urine to suitable urine collection means such as a conventional flexible collection bag.

The catheter assembly further comprises a flexible tubular catheter package including a hose member 4 providing a cavity 5 narrowly surrounding the catheter 1. At the first end of the hose member 4 a first detachable closure is provided which in the embodiment shown comprises a connector part 6 similar to a standard hose connector and inseparably connected with the first end of the hose member 4 and a break-off closure part 7. The connector part 6 may be profiled at its external side to provide a sealed connection with the connector member 3 of the catheter 1 and may be connected with the break-off closure part 7 through a notch-like incision 8 providing for easy breaking-off of closure part 7.

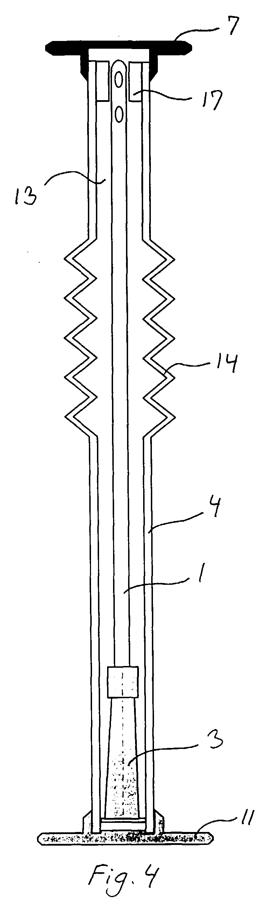

At its second end the hose member 4 is inseparably connected with an end member 9 which is detachably connected, e.g. by a notch-like incision in the same way as shown in Fig. 4, with a break-off compartment 10, in which the connector member 3 of the catheter 1 is accommodated. At its open end the compartment 10 is closed by a detachable cover 11.

The hose member shown in Fig. 1 is provided with elongation means in the form of click-clack corrugations 14 and soft corrugations 15. At the ends and in between the click-clack corrugations 14 and the soft corrugations 15 the hose member 4 has a smooth part 16.

In the embodiment shown in Fig. 1 the closure part 7 at the first end of the hose member 4 as well as the end member 9 and the break-off compartment 10 are provided with relatively large projecting gripping flanges 12 for facilitating operation of the catheter assembly by disabled users who may frequently suffer from a severely reduced dexterity.

In the illustrated preferred embodiment of the catheter assembly the catheter 1 is preferably of the kind known per se, which is provided throughout the length of the catheter shaft intended for insertion into the urethra with a hydrophilic surface coating requiring preparation with a liquid swelling medium prior to use in order to obtain a very slippery low-friction surface which is desirable for inserting the catheter without causing any discomfort to the user.

By use of the catheter assembly according to the invention execution of the necessary preparation of the hydrophilic surface in a separate operation is avoided by integration of the amount of liquid swelling medium needed for the preparation in the package. As shown in Figs. 1 and 4, a liquid 13 is confined in the relatively narrow cavity of the hose member 4 surrounding the catheter shaft.

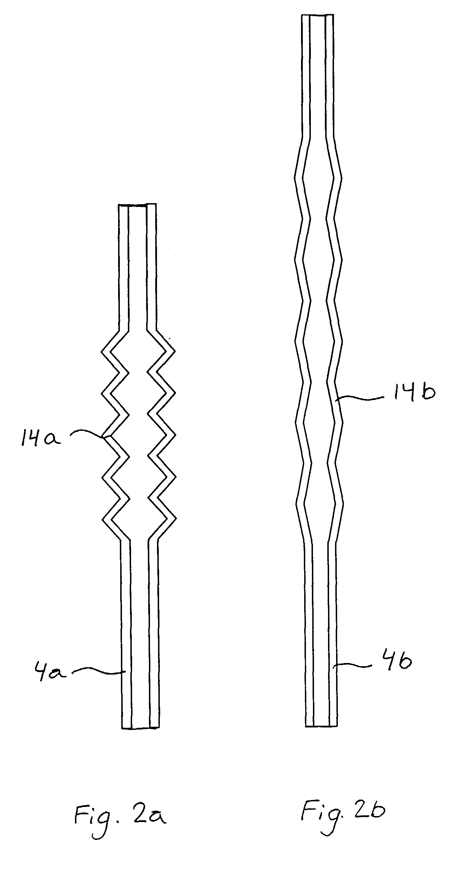

As shown in Fig. 2a, the hose member 4a is provided with click-clack corrugations 14a. When the click-clack corrugations are stretched 14b as shown in Fig 2b the hose member 4b is substantially longer than before stretching.

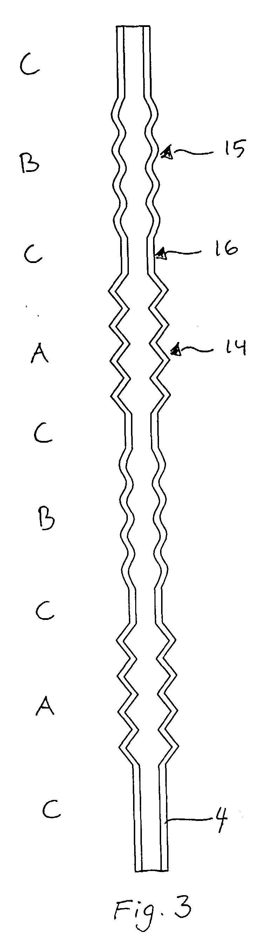

As illustrated in Fig. 3, the click-clack corrugations may be designated by the letter A, the soft corrugations by the letter B and the smooth part of the hose member 4 may be designated by the letter C. One can understand that there will be many possible combinations all covered by the present invention. Especially suitable combinations are however the following: C A C B C A C B C, C A B A B A B C, C A A A A C B B B B C, C A A A A B B B B C and C A A A A A A A A C. The combination C A A A A A A A A C = click-clack corrugations along substantially the whole length of the hose member 4 is especially suitable for female catheters as the hose member 4 for a female catheter assembly needs to be elongated more than a similar hose member 4 for a male catheter.

It is advantageous that the compartment 10 is detachably connected with the hose member 4 and is formed with walls of a thin flexible material to permit an easy arrangement of the compartment, after detachment and removal from the hose member 4, on the catheter 1 for use as an applicator for guided non-contaminating insertion of the catheter 1 into the urethra.

In yet a further preferred embodiment of the urinary catheter assembly according to the invention, a second end of the catheter is connected with a connector member 3, and the first end of the hose member 4, remote from the connector of the catheter, is closed by a first closure 7. The connector member 3 of the catheter has an outer diameter smaller than the inner diameter of the hose member 4 and may pass through the holes upon opening of the first closure 7 when extracting the catheter through the first end of the hose member, the first end of the hose member being provided with one or more stop members 17 as shown on Fig. 4, and that the stop members have a smaller internal opening than the external diameter of the connector member 3.

Again the catheter package acts as an extension for the catheter when the catheterization takes place, and the urine flows through the catheter and the catheter package directly into a toilet or other available drainage means without spillage, only the catheter and its package are left as items which must be disposed after use. The hose member in this embodiment may also be elongated by using the elongation means before the catheterization takes place. The catheter package therefore defines a substantially longer extension for the catheter.

The catheter assembly of the invention is suitable, in particular, for use with catheters having a hydrophilic surface coating throughout the part of their length intended for insertion into the urethra.

Thus, in a further preferred embodiment the catheter is provided on at least a part of its surface with a hydrophilic surface coating intended to produce a low-friction surface character of the catheter by treatment with a liquid swelling medium prior to use of the catheter, and the package includes an amount of the liquid swelling medium sufficient for the treatment of the hydrophilic catheter surface coating. Since the hose member used as a package for the catheter provides a cavity narrowly surrounding the catheter shaft, the amount of liquid swelling medium can be limited to what is needed for preparation of the hydrophilic coating.

By the integration of the amount of swelling medium needed for preparation of the hydrophilic surface coating in the catheter assembly, preparation of the hydrophilic surface coating is effected without separate treatment prior to use of the catheter. When removed from the hose member the catheter will be immediately ready for use and have the low-friction characteristics needed for its insertion into the urethra.

## Claims

1. A urinary catheter assembly comprising a urinary catheter (1) and a flexible tubular catheter package comprising a hose member (4) with a cavity narrowly surrounding the catheter (1), characterised in that at least part of the hose member (4) is provided with elongation means.

2. A urinary catheter assembly as claimed in claim 1, characterised in that the elongation means is in the form of click-clack corrugations (14).

3. A urinary catheter assembly as claimed in claim 1 or 2, characterised in that at least part of the hose member is provided with soft corrugations (15).

4. A urinary catheter assembly as claimed in any of the claims 1 to 3, wherein a first end of the catheter is connected with a connector member (3), wherein the package further comprises a tubular compartment (10) connected with said hose member (4) for accommodation of said connector member (3), wherein said compartment (10) is closed by a first closure (11), whereas in a second, opposite, end it is connected with the hose member (4), and wherein a second end of the hose member (4) remote from its connection with said compartment (10) is closed by a second closure (7), said connector member (3) being adapted for connection with said second end of the hose member (4) after removal of said first cover member (11) and said second closure (7), so that when removing the catheter (1) from the hose member (4) the latter forms an extension member in flow communication with the catheter (1).

5. A urinary catheter assembly as claimed in any of the claims 1 to 4, wherein the second end of the hose member (4) is inseparably connected with a hose connector (6) matching said connector member (3), said closure (7) being detachably connected with said hose connector (6).

6. A urinary catheter assembly as claimed in any of the claims 1 - 5, wherein the compartment (10) is detachably connected with said hose member (4) and is formed with walls of a thin flexible material to permit arrangement of said compartment (10), after detachment and removal from the hose member (4), on the catheter (1) for use as an applicator for guided non-contaminating insertion of the catheter (1) into the urethra.

7. A urinary catheter assembly as claimed in any of the claims 1 to 6, wherein a first end of the catheter is connected with a connector member (3), and the second end of the hose member (4), remote from the connector of the catheter, is closed by a second closure (7), and wherein the connector member (3) of the catheter has an outer diameter smaller than the inner diameter of the hose member (4), said second end of the hose member being provided with one or more stop members (17) at its second end, and wherein the stop members have a smaller internal opening than the external diameter of the connector member (3).

8. A urinary catheter assembly as claimed in any of the claims 1 to 7, wherein said catheter (1) is provided on at least a part of its surface with a hydrophilic surface coating intended to produce a low-friction surface character of the catheter by treatment with a liquid swelling medium prior to use of the catheter, and the package includes an amount of said liquid swelling medium sufficient for said treatment of the hydrophilic catheter surface coating .
